# EUROPEAN PATENT SPECIFICATION

(11) **EP 0 592 130 B1**
(45) Date of publication and mention of the grant of the patent: **14.04.1999**
(21) Application number: 93307598.8
(22) Date of filing: 24.09.1993
(51) Int. Cl.: A61K 9/48, A61J 3/07

(54) **Hard capsules**
Hartkapseln
Capsules dures

(30) Priority: 06.10.1992 JP 292094/92
(43) Date of publication of application: 13.04.1994
(73) Proprietor: Shionogi Qualicaps Co., Ltd., Yamatokoriyama-shi, Nara-ken (JP)
(72) Inventor: Yamamoto, Taizo, Osaka-shi, Osaka (JP); Matsuura, Seinosuke, Kizu-cho, Souraku-gun, Kyoto (JP); Hashimoto, Kiyoaki, Shiki-gun, Nara-ken (JP); Abe, Kenji, Shiki-gun, Nara-ken (JP); Akai, Kazukiyo, Kashihara-shi, Nara-ken (JP)
(74) Representative: Stoner, Gerard Patrick

(56) References cited:
- EP-A- 0 180 287
- EP-A- 0 246 693
- EP-A- 0 276 813
- PATENT ABSTRACTS OF JAPAN vol. 16, no. 98 (C-0918) 11 March 1992 & JP-A-03 279 325 (NIPPON ERANKO KK) 10 December 1991

## Description

This invention relates to hard capsules, especially intended for enclosing drugs therein.

### Prior Art

Conventional hard capsules to be filled with drugs are generally formed from shell-forming compositions comprising gelatin as a base, plasticizers such as glycerin and sorbitol, opacifying agents, dyes and pigments. Such gelatin shells tend to lose plasticity at a water content in excess of 10% by weight and hence their impact resistance lowers below the practical level required for drug filling operation. Conversely, if shells lower their water content during capsule storage, they will shrink so that the cap and body engagement becomes loose with the lapse of time. So, it is typically necessary to always keep the water content of hard gelatin capsule shells at a constant level of about 10 to 15% by weight. If capsule having such a water content is filled with drugs subject to hydrolysis, or two or more potentially interactive drugs, then the water content of the shell can have influences on the drugs, for example, a drop of drug potency, degradation, and discoloration. Sometimes the shell can be insolubilized.

Also known in the art are pharmaceutical hard capsules using bases other than gelatin. They are typically prepared using as a base water-soluble cellulose ether in which some or all of the hydroxyl groups of cellulose are replaced by alkyl groups or hydroxyalkyl groups, for example, by immersing shaping pins in an aqueous solution of the cellulose ether and forming shells around the pins as disclosed in Japanese Patent Publication (JP-B) No. 4310/1972, or by blending the water-soluble cellulose ether with polyvinyl alcohol and forming an aqueous solution of the blend for immersion as disclosed in Japanese Patent Application Kokai (JP-A) Nos. 100519/1986 and 266060/1987.

These capsule preparing methods generally include immersing shaping pins in an immersion solution of a water-soluble cellulose derivative as a base, forming coatings around the pins, and heating the coatings into gel for shaping of shells. If heating is insufficient, the base immersion solution is not gelled or solidified, permitting the coatings to slip or fall down from the shaping pins. Too high heating temperatures, on the other hand, will cause the gelled shells to crease. In particular, the latter method has a problem that when the water-soluble cellulose derivative adhered to the shaping pins is gelled by immersing in hot water, the gelled coatings will partially dissolve out into water. It is then difficult to obtain uniform shells. Due to low jelly strength, cracks are often introduced into the dry shells when they are released from the shaping pins. For these and other reasons, there are difficulties in using these methods to make practically acceptable pharmaceutical hard capsules with a limited low water content.

An additional drawback of these methods is that they require special apparatus and operation and cannot employ conventional gelatin capsule producing apparatus without substantial modifications.

To solve these problems, the inventors proposed in JP-A 279325/1991 a pharmaceutical hard capsule with a low water content comprising a water-soluble cellulose derivative as a base and a gelling agent and a co-gelling agent blended therewith. This hard capsule has the advantages that it exhibits equivalent properties to the conventional hard gelatin capsules, is of higher practical value, and can be prepared by a conventional method and apparatus for preparing hard gelatin capsules. However, it is somewhat inferior in outer appearance to the conventional hard gelatin capsules. There is room for improvement in this respect.

Making extensive investigations on this capsule for improving its outer appearance and color tone, we have found that when hydroxypropylmethyl cellulose (often abbreviated as HPMC, hereinafter) which has been exposed to ultraviolet light in the wavelength range of at least 200 nm is used as the water-soluble cellulose derivative base, there can be obtained hard capsules which have higher whiteness than conventional HPMC, maintain the inherent properties of conventional HPMC unchanged, have a low equilibrium water content, do not degrade at low humidity, are free of detrimental influences of water including cracking after drug filling, degradation and discoloration of drugs, and have acceptable solubility and mechanical strength of the capsule shell as well as good outer appearance.

We have also found that when purified carrageenan is used as the gelling agent in a pharmaceutical hard capsule of this type e.g. of JP-A 279325/1991, we can obtain capsules having good outer appearance but free of the unpleasant odor and taste inherent to carrageenan because purification removes soluble salts, coloring impurities and low molecular weight impurities from carrageenan. Especially when HPMC which has been exposed to ultraviolet light in the wavelength range of at least 200 nm is used as the base and purified carrageenan is used as the gelling agent, it is possible to make high quality hard capsules having a low water content. The present invention is predicated on these findings.

Therefore, the present invention is directed to a hard capsule comprising a water-soluble cellulose derivative as a base, a gelling agent and a co-gelling agent. In a first aspect, the water-soluble cellulose derivative is hydroxypropylmethyl cellulose which has been exposed to ultraviolet light in the wavelength range of at least 200 nm. In a second aspect, the gelling agent is purified carrageenan which is typically obtained by treating carrageenan with an aqueous solution of a potassium salt. In a preferred aspect, the water-soluble cellulose derivative is hydroxypropylmethyl cellulose which has been exposed to ultraviolet light in the wavelength range of at least 200 nm and the gelling agent is purified carrageenan.

Other aspects provide methods in which the capsules are prepared, using the irradiation of HPMC and/or the purified carrageenan. A further aspect is a drug capsule comprising the capsule containing a drug substance.

### DETAILED DESCRIPTION

The hard capsule of the present invention is formed of a composition including a water-soluble cellulose derivative as a shell forming base, a gelling agent and a co-gelling agent.

In the first aspect, the water-soluble cellulose derivative is hydroxypropylmethyl cellulose (HPMC) which has been exposed to ultraviolet light in the wavelength range of at least 200 nm. The HPMC used herein may be a conventional commercially available powder product, for example, TC-5R and TC-5RW commercially available from Shin-Etsu Chemical Co., Ltd.

With respect to the treatment of HPMC by light exposure, the light exposure time is not critical. Light exposure may be done on HPMC powder before or after capsule shaping, or at any intermediate stage. Light exposure may also be done in plural times at appropriate stages from the powder stock stage to the completion of capsule shaping. Light exposure conditions may be appropriately selected although it is preferred to select as the light source emitting ultraviolet light in the wavelength range of at least 200 nm a ultraviolet germicidal lamp, e.g. a lamp emitting ultraviolet radiation at 253.7 nm. Preferred conditions for ultraviolet radiation are a spacing of about 1 to 20 cm for about 1 to 20 hours. For white HPMC capsules, this ultraviolet exposure is effective for improving the whiteness thereof as compared with that before exposure. For colored HPMC capsules, the ultraviolet exposure is effective for making the color tone thereof clearer.

In preparing the capsules, HPMC powder is preferably blended in an amount of 5 to 25% by weight, especially 13 to 17% by weight, of an aqueous base solution from which capsules are formed. Less than 5% of HPMC powder makes it difficult to form capsule shells of sufficient gauge, whereas more than 25% of HPMC powder tends to provide a base with a higher jelly viscosity which obstructs shaping of uniform capsule shells by immersion.

In addition to the base defined above, the gelling and co-gelling agents are used in the hard capsule forming composition. The gelling agents include carrageenan, tamarind seed polysaccharides, pectin, curdlan, gelatin, phaselelan, and agar. Preferred among these is carrageenan since it has high gel strength and good gelling properties. While there are known three types of carrageenan, kappa-, iota- and lambda-carrageenans, the invention favors the use of kappa- and iota- carrageenans having gelling capability.

The co-gelling agents used herein vary with a choice of the gelling agent. The co-gelling agents used in combination with kappa-carrageenan include water-soluble compounds containing one or more of potassium, ammonium and calcium ions, for example, potassium chloride, potassium phosphate, calcium chloride, and ammonium chloride. The co-gelling agents used in combination with iota-carrageenan include water-soluble compounds containing a calcium ion, for example, calcium chloride.

The gelling agent may be blended in known amounts, preferably 0.1 to 0.5% by weight, especially 0.15 to 0.3% by weight of the aqueous base solution. With less than 0.1% of the gelling agent, the aqueous base solution adhered to shaping pins during immersion shaping typically may not gel, and slip down from the pins. A base solution containing more than 0.5% of the gelling agent would usually have too high a jelly viscosity to form uniform capsule shells by immersion techniques. In addition, it tends to form a gel film on the wall of an immersion solution container, causing obstructions during capsule shell shaping.

The co-gelling agent is preferably blended in an amount of 0.01 to 0.5% by weight, especially 0.05 to 0.2% by weight of the aqueous base solution. Outside the range, there may arise inconvenient problems as with the gelling agent.

Like conventional hard capsules, the capsules of the invention may contain in addition to the above-mentioned essential ingredients, any desired additives such as coloring agents (dyes and pigments), opacifying agents and flavors insofar as the objects of the invention are not impaired.

Like conventional hard capsules, the capsules of the invention may be prepared by a conventional immersion shaping technique. At first, HPMC, gelling agent, co-gelling agent and other desired additives are appropriately blended to form an aqueous base solution. Shaping pins are immersed in the aqueous base solution. At this point, the aqueous base solution or immersion solution is preferably adjusted to a temperature of 48 to 55°C, especially 50 to 52°C. Outside the range, the immersion solution tends to undergo more or less changes in jelly viscosity, and the immersion solution may then adhere poorly to the shaping pins during immersion shaping, leading to difficult formation of uniform capsule shells.

Then the shaping pins are withdrawn from the immersion solution. Subsequent steps of drying, releasing the shells from the shaping pins, and cutting yield hard capsule shells of predetermined dimensions. These steps may be carried out in the same manner as used for conventional hard gelatin capsules, Usually it takes 30 to 60 seconds until the base immersion solution gels on the shaping pin surface.

According to the first aspect of the invention, HPMC is exposed to ultraviolet light in the wavelength range of at least 200 nm at any (one or more) step in the above-mentioned process whereby there are obtained hard capsules containing light irradiated HPMC as the base.

The resulting hard capsules generally contain 92 to 94% by weight of HPMC, 0.9 to 1.2% by weight of the gelling agent, and 0.5 to 0.6% by weight of the co-gelling agent. They generally have a water content of 4 to 6% by weight.

In the second aspect, the hard capsule is formed of a composition including a water-soluble cellulose derivative as a shell forming base, a gelling agent and a co-gelling agent wherein the gelling agent is purified carrageenan.

The carrageenan used herein is as previously described and is purified, preferably by simply washing carrageenan with an aqueous solution of a potassium salt for thereby removing soluble salts, coloring impurities and low molecular weight impurities from the carrageenan. The aqueous potassium salt solution used for washing is typically an aqueous solution of potassium chloride or potassium phosphate in a concentration of 1 to 15% by weight, preferably 8 to 12% by weight. It would be difficult to uniformly disperse carrageenan powder in a solution at a potassium salt concentration of less than 1% whereas at concentrations of more than 15%, excess potassium chloride will remain on the carrageenan powder after purification to prevent adequate gelation of carrageenan. Washing with a potassium salt solution is preferably carried out three or more times, preferably three to five times because less than three times of washing could sometimes leave slight unpleasant odor or bitterness.

The water-soluble cellulose derivatives used herein are preferably cellulose ethers in which some or all of the hydroxyl groups of cellulose are replaced by alkyl groups, especially lower alkyl groups and/or hydroxy lower alkyl groups. Examples include hydroxypropylmethyl cellulose, hydroxyethyl cellulose, hydroxypropyl cellulose, and hydroxyethylmethyl cellulose, with the hydroxypropylmethyl cellulose being most preferred from the standpoints of shell forming ability and mechanical strength at low water contents.

The preferred amount of water-soluble cellulose derivative, the amount of purified carrageenan, the type and amount of the co-gelling agent, and the hard capsule preparing process are the same as in the first aspect. In the second aspect, hard capsules may be prepared without light exposure.

In the third aspect, the water-soluble cellulose derivative is light irradiated HPMC as defined in the first aspect and the gelling agent is purified carrageenan as defined in the second aspect. The preferred amounts of the ingredients and the hard capsule preparing process are the same as in the first aspect.

Hard capsules made in this way may be useful not only in the pharmaceutical field for enclosing drugs, but also in food and other fields.

The hard capsules of the first aspect have good outer appearance since light irradiated HPMC has higher whiteness than conventional HPMC. Since light irradiated HPMC maintains the inherent properties of conventional HPMC unchanged, the capsules have a low equilibrium water content, do not become brittle at low humidity, are free of detrimental influences of water including cracking after drug filling, degradation and discoloration of drugs, and have acceptable solubility and mechanical strength of the capsule shell. Where purified carrageenan is blended as the gelling agent, the hard capsules have aesthetic outer appearance and are substantially free of unpleasant odor and taste and thus suitable for oral administration.

### EXAMPLE

Examples of the present invention are given below by way of illustration and not by way of limitation.

### Examples 1-3 & Comparative Example 1

In 19.55 liters of pure water at about 70°C was dissolved 18.4 grams of potassium chloride (co-gelling agent concentration 0.08% by weight). With stirring, 39.1 grams of kappa-carrageenan (gelling agent concentration 0.17% by weight) was added to and dissolved in the solution.

With stirring, 3.45 kg of hydroxypropylmethyl cellulose (cellulose derivative concentration 15% by weight) was added to the solution and dispersed in hot water. The temperature of the dispersion was lowered to 50°C and agitation was continued to dissolve the hydroxypropylmethyl cellulose in water. The solution was allowed to stand for 7 hours and then deaerated. An immersion solution or aqueous base solution was obtained in this way.

A well-known capsule preparing apparatus based on an immersion technique was charged with the immersion solution. While the immersion solution was kept at a temperature of 50 to 52°C, white hard capsules of No. 1 size were prepared in a conventional manner.

The white HPMC capsules of No. 1 size were exposed to light from a white fluorescent lamp or UV germicidal lamp under the following conditions. Thereafter, the capsules were measured for whiteness using a spectral colorimeter manufactured by Juki K.K. The results are shown in Table 1.

### Exposure conditions

| | |
|---|---|
| Exposure atmosphere | room temperature |
| Lamp-capsule spacing | 14 cm |
| White fluorescent lamp | 18 W |
| UV germicidal lamp | 10 W |

**Table 1**

| | Irradiating light (time) | Whiteness |
|---|---|---|
| Comparative Example 1 | no exposure | 87.37 |
| Example 1 | white fluorescent lamp (1 hour) | 88.16 |
| Example 2 | UV germicidal lamp (1 hour) | 88.98 |
| Example 3 | UV germicidal lamp (10 hours) | 89.63 |

As seen from Table 1, light irradiated HPMC capsules are significantly improved in whiteness as compared with non-irradiated HPMC capsules.

### Examples 4-7 & Comparative Examples 2-3

Two commercial HPMC powders were exposed to light under the same conditions as Examples 2 and 3 and measured for whiteness. The results are shown in Table 2.

**Table 2**

| | HPMC powder* | Irradiating light (time) | Whiteness |
|---|---|---|---|
| Comparative Example 2 | Standard | no exposure | 91.03 |
| Example 4 | Standard | UV germicidal lamp (1 hour) | 91.35 |
| Example 5 | Standard | UV germicidal lamp (10 hours) | 93.33 |
| Comparative Example 3 | Improved | no exposure | 92.50 |
| Example 6 | Improved | UV germicidal lamp (1 hour) | 92.62 |
| Example 7 | Improved | UV germicidal lamp (10 hours) | 93.61 |

| | | | |
|---|---|---|---|
| *"Standard" is a commercial HPMC powder of the standard type available under the trade name TC-5F from Shin-Etsu Chemical Co., Ltd. "Improved" is a commercial HPMC powder of a whiteness-improved type available under the trade name TC-5RW from Shin-Etsu Chemical Co., Ltd. | | | |

As seen from Table 2, UV irradiated HPMC powders are significantly improved in whiteness as compared with non-irradiated HPMC powders. This indicates that exposure of HPMC in powder form to UV is effected.

### Measurement

The HPMC capsules of Example 3 and Comparative Example 1 were measured for solubility, equilibrium water content and mechanical strength by the following methods. The results are shown in Table 3.

### Solubility

According to the Pharmacopeia of Japan, the official dissolving and disintegrating tests were carried out. The official dissolving test used 5 empty capsules. Reported is the time taken until the capsule was dissolved out. The official disintegrating test used 6 capsules filled with corn. Reported are the time taken until the capsule was opened (Open) and the time taken until the entire contents had flown out of the capsule (Flowout).

### Equilibrium water content

Capsules were allowed to stand for 14 days at a relative humidity of 43% and 57% in a desiccator where containers of a saturated potassium carbonate solution and a saturated sodium bromide solution were placed. The capsules were taken out and measured for water content by a loss on drying test (105°C, 2 hours).

### Mechanical strength

Impact resistance was determined by a free falling dart test in which an empty capsule was laid on a platform and a dart of 50 grams was dropped from a height of 10 cm. The results is expressed by the number of broken samples per 50 test samples.

**Table 3**

| | Comparative Example 1 | | Example 3 | |
|---|---|---|---|---|
| | non irradiated | | UV lamp (10 hours) | |
| | | | | |

| Official dissolving test (5 empty capsules) | | | | |
|---|---|---|---|---|
| Average | 9′10˝ | | 8′50˝ | |
| Maximum | 9′34˝ | | 9′27˝ | |
| Minimum | 8′40˝ | | 7′56˝ | |

| Official disintegrating test (6 corn-filled capsules) | | | | |
|---|---|---|---|---|
| | Open | Flowout | Open | Flowout |
| Average | 2′40˝ | 3′51˝ | 2′43˝ | 3′48˝ |
| Maximum | 3′56˝ | 5′05˝ | 3′58˝ | 5′00˝ |
| Minimum | 1′55˝ | 3′00˝ | 2′00˝ | 3′05˝ |

| Equilibrium water content | | | | |
|---|---|---|---|---|
| at RH 43% | 5.2% | | 5.2% | |
| at RH 57% | 7.8% | | 7.7% | |
| Impact resistance | 0/50 (water content 1.4%) | | 0/50 (water content 1.4%) | |

As seen from Table 3, UV irradiated HPMC capsules embodying the invention were of good quality, having properties equivalent to non-irradiated HPMC capsules including a low equilibrium water content and satisfactory solubility and mechanical strength.

### Example 8-12

Commercially available carrageenan (manufactured by Sansho K.K.) was purified by washing it with an aqueous solution of 10% potassium chloride plural times (shown in Table 4) followed by filtration. Hard capsules were prepared by the same procedure as in Examples 1-3 except that the purified carrageenan was used. The capsules were examined for odor and taste by an organoleptic test. The results are shown in Table 4.

**Table 4**

| | Washing time | Organoleptic test | |
|---|---|---|---|
| | | Odor | Taste |
| Control | - | unpleasant odor | bitter |
| Example 8 | 1 | slight unpleasant odor | slight bitter |
| Example 9 | 2 | slight unpleasant odor | slight bitter |
| Example 10 | 3 | no unpleasant odor | not bitter |
| Example 11 | 4 | no unpleasant odor | not bitter |
| Example 12 | 5 | no unpleasant odor | not bitter |

As seen from Table 4, HPMC capsules having purified carrageenan blended are of good quality, being substantially free of the unpleasant odour and taste normally inherent to carrageenan.

### Example 13

White hard capsules of No. 1 size were prepared by the same procedure as in Examples 1-3 except that the purified carrageenan of Example 10 was used, and then exposed to UV radiation under the same conditions as in Example 3.

The resulting capsules had very high whiteness and were free of unpleasant odor and bitterness.

## Claims

1. A hard capsule formed from a capsule composition comprising water-soluble cellulose derivative as a base ingredient blended with a gelling agent and a co-gelling agent, characterised in that the cellulose derivative is hydroxypropyl methylcellulose (HPMC) which has been subjected to treatment with ultraviolet light of at least 200nm wavelength.

2. A capsule according to claim 1 in which the ultraviolet light has 253.7nm wavelength.

3. A capsule according to claim 1 or claim 2 in which for said treatment the HPMC is spaced at from 1 to 20cm from the ultraviolet source, for a period of from 1 to 20 hours.

4. A capsule according to any one of the preceding claims in which the gelling agent is selected from carrageenan, tamarind seed, polysaccharides, pectin, curdlan, gelatin, phaselelan and agar.

5. A capsule according to claim 4 in which the gelling agent is purified carrageenan.

6. A capsule according to claim 5 in which the carrageenan has been purified by treatment with aqueous solution of potassium salt.

7. A capsule according to any one of claims 4 to 6 in which the gelling agent is kappa-carrageenan and the co-gelling agent is selected from water-soluble ionic compounds of potassium, ammonium and calcium, or in which the gelling agent is iota-carrageenan and the co-gelling agent is selected from water-soluble ionic calcium compounds.

8. A capsule according to any one of the preceding claims containing 92 to 94 wt% HPMC, 0.9 to 1.2% gelling agent, 0.5 to 0.6% co-gelling agent and 4 to 6 wt% water.

9. A method of making hard capsules as defined in any one of claims 1 to 8, comprising blending the HPMC, gelling agent and co-gelling agent to form an aqueous base solution, immersing shaping pins in the aqueous base solution, withdrawing them; drying, releasing and cutting the formed capsule shells,
and including subjecting the HPMC to said treatment with ultraviolet light at one or more stages of the method.

10. A method according to claim 9 in which the HPMC is subject to the ultraviolet treatment before the shaping of the capsules.

## Patentansprüche

1. Hartkapsel, die aus einer Kapselzusammensetzung gebildet ist, die wasserlösliches Cellulosederivat als Basisbestandteil, vermischt mit einem Geliermittel und einem Cogeliermittel umfaßt, dadurch gekennzeichnet, daß das Cellulosederivat Hydroxypropylmethylcellulose (HPMC) ist, die einer Behandlung mit UV-Licht mit einer Wellenlänge von zumindest 200 nm unterzogen wurde.

2. Kapsel nach Anspruch 1, worin das UV-Licht eine Wellenlänge von 253,7 nm aufweist.

3. Kapsel nach Anspruch 1 oder 2, worin die HPMC zur Behandlung 1 bis 20 h lang in einem Abstand von 1 bis 20 cm von der UV-Lichtquelle entfernt angeordnet ist.

4. Kapsel nach einem der vorangegangenen Ansprüche, worin das Geliermittel aus Carrageenan, Tamarinden-Samen, Polysacchariden, Pektin, Curdlan, Gelatine, Phaselelan und Agar ausgewählt ist.

5. Kapsel nach Anspruch 4, worin das Geliermittel gereinigtes Carrageenan ist.

6. Kapsel nach Anspruch 5, worin das Carrageenan durch Behandlung mit einer wäßrigen Kaliumsalzlösung gereinigt wurde.

7. Kapsel nach einem der Ansprüche 4 bis 6, worin das Geliermittel κ-Carrageenan ist und das Cogeliermittel aus wasserlöslichen ionischen Kalium-, Ammonium- und Calciumverbindungen ausgewählt ist, oder worin das Geliermittel ℩-Carrageenan ist und das Cogeliermittel aus wasserlöslichen ionischen Calciumverbindungen ausgewählt ist.

8. Kapsel nach einem der vorangegangenen Ansprüche, die 92 bis 94 Gew.-% HPMC, 0,9 bis 1,2 % Geliermittel, 0,5 bis 0,6 % Cogeliermittel und 4 bis 6 Gew.-% Wasser enthält.

9. Verfahren zur Herstellung von Hartkapseln nach einem der Ansprüche 1 bis 8, umfassend das Vermischen von HPMC, Geliermittel und Cogeliermittel, um eine wäßrige Basislösung zu bilden, das Eintauchen von Formzapfen in die wäßrige Basislösung, Herausziehen derselben; das Trocknen, Loslösen und Schneiden der gebildeten Kapselhüllen,
sowie umfassend das Unterziehen der HPMC der Behandlung mit UV-Licht in einem oder mehreren Verfahrensschritten.

10. Verfahren nach Anspruch 9, bei dem die HPMC vor dem Formen der Kapseln der UV-Behandlung unterzogen wird.

## Revendications

1. Capsule dure formée d'une composition pour capsule comprenant un dérivé de cellulose soluble dans l'eau en tant qu'ingrédient de base mélangée avec un agent de gélification et un agent de co-gélification, caractérisée en ce que le dérivé de cellulose est de l'hydroxypropyl méthylcellulose (HPMC) qui a été soumise à un traitement avec une lumière ultraviolette d'au moins 200 nm de longueur d'onde.

2. Capsule selon la revendication 1 dans laquelle la lumière ultraviolette a une longueur d'onde de 253,7 nm.

3. Capsule selon la revendication 1 ou la revendication 2 dans laquelle pour ledit traitement l'HPMC est placée à une distance de 1 à 20 cm de la source d'ultraviolets, pendant une période de 1 à 20 heures.

4. Capsule selon l'une quelconque des revendications précédentes dans laquelle l'agent de gélification est sélectionné parmi de la carragheen, un graine de tamarin, des polysaccharides, de la pectine, du curdlan, de la gélatine, du phasélan et de l'agar.

5. Capsule selon la revendication 4 dans laquelle l'agent de gélification est de la carragheen purifiée.

6. Capsule selon la revendication 5 dans laquelle la carragheen a été purifiée par traitement avec une solution aqueuse de sel de potassium.

7. Capsule selon l'une quelconque des revendications 4 à 6 dans laquelle l'agent de gélification est la kappa-carragheen et l'agent de co-gélification est sélectionné parmi des composés ioniques solubles dans l'eau de potassium, ammonium et calcium, ou dans laquelle l'agent de gélification est la iota-carragheen et l'agent de co-gélification est sélectionné parmi des composés de calcium ioniques solubles dans l'eau.

8. Capsule selon l'une quelconque des revendications précédentes contenant 92 à 94% en poids d'HPMC, 0,9 à 1,2% d'agent de gélification, 0,5 à 0,6% d'agent de co-gélification et 4 à 6% d'eau.

9. Méthode de fabrication de capsules dures telles que définies dans l'une quelconque des revendications 1 à 8, comprenant le mélange de l'HPMC, de l'agent de gélification et de l'agent de co-gélification pour former une solution de base aqueuse, l'immersion d'aiguilles de mise en forme dans la solution de base aqueuse, leur retrait; le séchage, la libération et la coupe des coquilles de capsules formées,
et incluant la soumission de l'HPMC audit traitement avec la lumière ultraviolette à une ou plus étape de la méthode.

10. Méthode selon la revendication 9, dans laquelle l'HPMC est soumise au traitement avec des ultraviolets avant la mise en forme des capsules.
